(19) **Europäisches Patentamt**

**European Patent Office**

**Office européen des brevets**

(11) **EP 1 649 829 A1**

(12) **EUROPEAN PATENT APPLICATION**

(43) Date of publication:
**26.04.2006 Bulletin 2006/17**

(51) Int Cl.:
**A61F 2/06** (2006.01)

(21) Application number: **05022438.5**

(22) Date of filing: **14.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **14.10.2004 US 965590**

(71) Applicant: **Murphy, Kieran P.
Baltimore, MD 21210 (US)**

(72) Inventor: **Murphy, Kieran P.
Baltimore, MD 21210 (US)**

(74) Representative: **Körber, Martin Hans et al
Mitscherlich & Partner,
Patent- und Rechtsanwälte,
Sonnenstrasse 33
80331 München (DE)**

(54) **Stent delivery system with a balloon for a self-expandable stent**

(57)     The invention relates to stent delivery system (500) and method. A stent delivery system includes a self-expandable stent (501) and a sheath (502) configured to surround the stent in a compressed state. A catheter (504) is adapted to deliver the stent and the sheath into a predetermined deployment site. A balloon (503) is mounted on a tip of the catheter and is inflated to expand the stent. Upon deployment of the stent, the sheath is trapped between the stent and a vessel.

# FIG. 14

EP 1 649 829 A1

**Description**

PRIORITY CLAIM

The application is a continuation-in-part of Application Serial No. 10/727,667 filed December 5, 2003, which is a continuation-in-part of Application Serial No. 10/394,007 filed March 24, 2003. All of the foregoing applications are hereby incorporated by reference.

BACKGROUND

1. Technical Field

The invention relates to stent delivery system and method using a balloon for a self-expandable stent.

2. Background Information

Stroke and cardiac disease remain a major cause of morbidity and result in profound suffering and expense. Increased awareness and improvements in diagnostic procedures have significantly increased the diagnosis of cervical and intracranial and cardiac vascular stenosis. A vascular stenosis is now being treated endovascularly at a significantly increased frequency. However, follow-up has predominantly been by angiography which evaluates the vascular contour but not the vascular wall. It is invasive, time consuming and expensive. Preliminary studies suggest that stent evaluation and restenosis pathophysiology can also be evaluated with Multi-detector Computed Tomography Angiography ("MDCTA") which would be a significant advantage of this technique over conventional angiography.

More specifically, endovascular therapy has ushered in a new age of minimally invasive vascular treatment. Endovascular devices have been rapidly developed and refined. Present technologies have enabled precise deployment of stents in much smaller arteries and have become more flexible and compliant so they can be navigated through tortuosities. At the same time there has been a growing pool of physicians trained in modern endovascular therapies so services are more widely available. However, the monitoring of these patients has become suboptimal because it relies on conventional angiography which is invasive and expensive. It also requires the patient to spend a full day removed from their daily activities. It also requires that some patients on anticoagulation briefly discontinue their therapy or be admitted to the hospital for an extended period of time. New MDCTA technology has not been widely used or validated for follow up. However, preliminary case studies seem to indicate that this technology is likely to provide additional beneficial information about the vascular wall and stent not obtainable from conventional angiograms. MDCTA is also non-invasive, requires a minimal amount of time and is less costly. MDCTA now has an axial resolution less than 0.5 mm and with the proposed development of new protocols and algorithms for image processing, this will be a superior tool to evaluate stenting and the etiology of any restenosis or stent failures. In particular, it will likely be able to separate negative remodeling from neointimal growth. It will also be able to evaluate for stent deformity and wall apposition as well as remodeling. MDCTA should also be applicable to other endovascular procedures such as follow up for aneurysm coilings.

Indeed MDCTA reflects a number of advances in medical imaging that allow real time and/or three-dimensional image gathering under Computed Tomography ("CT"), Magnetic Resonance Imaging ("MRI") or the like. For example, CT scanners such as the Toshiba Acquillion multi detector are capable of generating images in three different areas at frame rates of 13 frames a second, to thereby generate a three-dimensional rendering of the target area. Indeed, this and other advances in CT have led to the development of new CT applications including CT Angiography ("CTA"), and CT Perfusion ("CTP"). These imaging modalities are rapidly developing into powerful tools in the diagnosis and treatment of both ischemic and hemorrhagic stroke and bilary occlusion. See, for example, the following prior art references:

Kopp AF, Ohnesorge B, Flohr T, Georg C, Schroder S, Kuttner A, Martensen J, Claussen CD. [Cardiac multidetector-row CT: first clinical results of retrospectively ECG-gated spiral with optimized temporal and spatial resolution]Rofo Fortschr Geb Rontgenstr Neuen Bildgeb Verfahr. 2000 May;172(5):429-35.

Ohnesorge B, Flohr T, Becker C, Knez A, Kopp AF, Fukuda K, Reiser MF. [Cardiac imaging with rapid, retrospective ECG synchronized multilevel spiral CT]Radiologe. 2000 Feb;40(2):111-7

Achenbach S, Moshage W, Ropers D, Nossen J, Bachmann K. Non-invasive coronary angiography with electron beam tomography: methods and clinical evaluation in post-PTCA follow-up Z Kardiol. 1997 Feb;86(2):121-30.

Becker CR, Schoepf UJ, Reiser MF.Methods for quantification of coronary artery calcifications with electron beam and conventional CT and pushing the spiral CT envelope: new cardiac applications.Int J Cardiovasc Imaging. 2001 Jun;17(3):203-11.

Kopp AF, Schroeder S, Kuettner A, Baumbach A, Georg C, Kuzo R, Heuschmid M, Ohnesorge B, Karsch KR, Claussen CD. Non-invasive coronary angiography with high resolution multidetector-row computed tomography. Results in 102 patients.Eur Heart J. 2002 Nov;23(21):1714-25.

Achenbach S, Ulzheimer S, Baum U, Kachelriess M, Ropers D, Giesler T, Bautz W, Daniel WG, Kalender WA, Moshage W. Non-invasive coronary angiography by retrospectively ECG-gated multislice spiral CT.Circulation. 2000 Dec 5;102(23):2823-8.

Knez A, Becker A, Becker C, Leber A, Boekstegers P, Reiser M, Steinbeck G. [Detection of coronary calcinosis with multislice spiral computerized tomography: an alternative to electron beam tomographyZ Kardiol. 2002 Aug;91 (8):642-9.

Mahnken AH, Sinha AM, Wildberger JE, Krombach GA, Schmitz-Rode T, Gunther RW. [The influence of motion artifacts conditioned by reconstruction, on the coronary calcium score in multislice spiral CT] Rofo Fortschr Geb Rontgenstr Neuen Bildgeb Verfahr. 2001 Oct;173(10):888-92.

However, despite these advances in medical device technology, and in particular stent technology and imaging technology, prior art stent technologies have certain limitations when viewed under such CT machines, particularly due to beam hardening artefacts that are typically present, which thereby obscure the image and obviate or reduce the effectiveness of the CT machine as a post-operative diagnostic tool.

Due to these present limitations using MDCTA, it is common to rely on classical angiography for postoperative evaluation of endovascular procedures, yet such angiographic methods are invasive and expensive. In the USA, an angiogram can cost up to $8000.00, yet a corresponding MDCTA could be offered for as little as $400.00. Additionally, endovascular ultrasound has significant associated risks and is not suitable for the small intracranial vessels. In the end, it is believed that MDCTA has the potential to provide good visualization of the lumen as well as the arterial wall and stent. MDCTA actually visualizes the stent better than fluoroscopy and will likely prove to be the preferred technique when background subtraction is used to increase vascular conspicuity. It is also believed that MDCTA would also enable more precise outcome evaluation and allow for investigation of the underlying pathophysiology as well as evaluation of the stents and devices used.

Polymer or lipid based drug delivery systems that can deliver drugs at a defined rate for up to five years from a single treatment have revolutionized medical therapy. Drug coated coronary stents have been shown to decrease restenosis rates in large clinical trials. See for example, the following references:

"Sirilimus eluting stents versus standard stents in patients with stenosis of the coronary artery", Moses *et al.* New England Journal of Medicine, page 1315-1323 October 2, 2003 Vol. 349, No. 14.

"Paclitaxel stent coating inhibits meointimal hyperplasia at 4 weeks in a porcine model of restenosis", Heldman *et al.* circulation 2001, 103-2289-95.

"A Paclitaxel eluting stent for the prevention of coronary restenosis", Park *et al.* New England Journal of Medicine 2003, Vol. 348, page 1537-45.

With respect to the drug delivery systems there are several types available at this time. These are principally those that are biodegradeable or those that are nonbiodegradeable. Biodegradable polymers release their loaded agents as they break down, while the matrix of non-biodegradable polymers remains intact even after all of the therapeutic agent has been released. These polymers release their loaded material by a process of either bulk erosion or surface erosion and diffusion or degradation. The polymers and co-polymers that are available at the present time include ethylene vinyl acetate ("EVAc"), a hydrophilic non biodegradable polymer, and biodegradeable polymers such as hydrophobic polymers such as poly[BIS (p-carboxyphenoxy)]propane-sebacic acid ("PCPP:SA"), hydrophilic polymers and fatty acid dimer-sebacic acid ("FAD:SA") polymers that deliver drugs including hydrophilic drugs and compounds.

A process such as lyophilization can be used to load the polymer with the desired compound or drug or compounds or drugs. In this was PCPP:SA, a desired compound such as iodinated contrast material, and methyl chloride may undergo the lyophilization process to load the PCPP:SA with a material with the ability to attenuate x-ray radiation and be visible on a radiographic image.

As previously stated, drug coated stents contribute to a reduction of restenosis. Nevertheless, when drug coated stents are deployed with conventional stent delivery systems, such effect of reducing restenosis may be undermined. Traditionally, conventional stent delivery systems generally use either a balloon-expandable stent or a self-expandable stent Typically, stent delivery systems are delivered to a predetermined deployment site, for example a stenosed area of a blood vessel by a catheter. Balloon-expandable stents are usually expanded at the deployment site by inflation of a balloon mounted on the tip of the catheter. After the stent has been expanded, the balloon is deflated and the catheter and balloon are removed from the deployment site. By contrast, self-expandable stents are capable of expanding without a balloon. Self-expandable stents are usually made of a spring metal, such as nitinol or stainless steel. Self-expandable stents are compressed onto the core of a catheter and retained by a sheath of the catheter. Once delivered to the deployment site, the self-expandable stent is expanded by retracting the sheath rearward, thereby releasing the stent. The self-expandable stent then expands against the vessel wall.

Unlike self-expandable stents, balloon-expandable stents are generally made of ductile material. Thus, balloon-expandable stents are more suitable for coronary arteries where external traumas usually do not reach the stent. In contrast, any deformation of a self-expandable stent is temporary because the stent returns to its original shape once the pressure is removed. Thus, self-expandable stents generally act like a spring and have a shape memory feature. Accordingly, self-expandable stents are more suitable for peripheral vessels including carotid arteries.

Regardless of the type of stent used, the stent implantation using conventional stent delivery systems involves multiple passages through the host blood vessel. The number of passages of the balloon, the protection device and the stent delivery system through the host vessel usually amounts to about five passes. Typically, a balloon is often inserted into the stent deployment site prior to the stent implantation to predilate the vessel. In addition, protection devices or filters are generally introduced into the vessel at the distal side of the deployment site to trap debris that may be released during the stent deployment. For instance, during carotid stenting, debris is substantially released at the time of (1) passage of the protection device across the lesion, (2) deployment of the stent, (3) the post stenting angioplasty, and (4) the removal of the protection device. Typically, the largest burst amount of debris release occurs at the time of passage of the balloon and protection devices. Debris released from the stent deployment may lead to embolization, which causes blockage of an artery or a vein. If the embolization results in blocking of blood flow in the heart, brain, liver, lungs or kidney, it can be critical.

As noted above, protection devices have been used to prevent or reduce embolization of debris released during stent deployment. Protection devices are typically disposed at a distal end of a catheter and released on the distal side of the deployment site in order to prevent distal flow of released debris, which later causes embolization. However, protection devices have not sufficiently shown the anticipated effects, i.e., reducing embolization, to justify the costs of inserting the protection devices. In particular, there is a risk that the protection device may cause a stroke during removal of the device. Further, where protection devices are used with the carotid vessel of a patient's brain, he or she often experiences traumatic feelings.

Accordingly, there is a need for a stent delivery system that overcomes the drawbacks of conventional stent delivery systems. Specifically, a stent delivery system allowing a postoperative evaluation under an imaging beam such as in a CT system is needed. In addition, a stent delivery system which can minimize embolic releases at the time of implanting a stent is needed.

BRIEF SUMMARY

In a first aspect of the invention there is provided a medical device made from a material operable to perform a therapeutic function of the device and wherein the material allows three-dimensional visualization of a surrounding tissue when the medical device is inserted into the tissue and viewed under an imaging beam.

It is therefore an object of the invention to provide a medical device that is viewable under certain imaging beams that obviates or mitigates at least one of the above-identified disadvantages of the prior art.

The medical device can be a stent and the surrounding tissue can be a lumen of a blood vessel. The stent can have a coating of a radiopaque material prior to insertion such that the stent that can be viewed during a conventional angiographic x-ray DA/DSA insertion and wherein the coating diminishes after insertion such that the stent can be viewed under CT post insertion. The stent can be coated with at least one of an antibiotic and a chemotherapy drug. The stent can be coated with at least one drug selected from the group consisting of a drug that is therapeutically effective to decrease attachment of platelets to the stent and a drug that is therapeutically effective to decrease restenosis. The drug can be selected from the group consisting of aspirin, plavix or paclitaxel.

In a particular implementation of the first aspect, the device can be selected from the group of devices for the treatment of obstruction due to clot, plaque, atheroma, tumours, and treatments involving intimal hyperplasia and recurrent stenosis.

The material used to manufacture the medical device can be selected from the group consisting of plastic, composite carbon fiber and Inconel, nitinol, stainless steel, or a radiolucent material.

The imaging system can be a substantially real-time CT machine, such as the Toshiba Acquillon.

The medical device can have an image density of less than about 1200 Hounsfield Units. The image density can be less than about 900 Hounsfield Units. The image density can be less than about 700 Hounsfield Units. The image density can be less than about 400 Hounsfield Units.

The medical device can be a microcoil and the surrounding tissue is an aneurysm repaired with the microcoil.

The configuration and structure of the medical device can be chosen to combine with the properties of the chosen material to provide a reduced beam hardened artifact. For example, where the device is a stent and the struts of the stent can be aligned or otherwise configured to reduce the beam hardened artifact.

In another aspect of the invention, a stent delivery system of a self-expandable stent mounted on a balloon is provided.

The stent delivery system comprises a self-expandable stent expanding from a compressed state to an expanded state and a sheath configured to constrain the stent having the compressed state and release the stent having the expanded state. The stent and the sheath are delivered by a catheter into a predetermined deployment site. The catheter also includes a balloon mounted on its tip and the balloon inflates to expand the stent. The sheath is fractured to release the stent. Alternatively, the sheath may be expanded along with the stent rather than fractured. In another embodiment, a sheath may be made of SIS (Small Intestine Submucosa) material.

In another aspect of the invention there is provided an imaging processing unit for a CT machine comprising:

a means for receiving multi-plane images of mammalian tissue;
a database of known medical devices and associated properties of the devices;
a means for determining whether an object detected in the received images matches with a known medical device in the database, the means for determining based on the associated properties;
means for applying a filter to the received images to enhance an image of the tissue that surrounds the implanted medical device based on the known associated properties; and,
means for presenting the image on an output device.

The database of known medical devices can include at least one of a stent and a microcoil. The associated properties in the database can include a Hounsfield unit measurement of the device.

BRIEF DESCRIPTION OF THE DRAWINGS

Embodiments of the invention will now be discussed, by way of example only, with reference to the attached Figures, in which:

Figure 1 is a representation of an imaging system;

Figure 2 is a side view of a prior art stent;

Figure 3 is a representation of a beam hardened artifact caused by the prior art stent of Figure 2 when viewed under the imaging system of Figure 1;

Figure 4 shows the beam hardened artifact of Figure 3 at a different angle;

Figure 5 shows the beam hardened artifact of Figure 4 at a different angle;

Figure 6 a side view of a stent in accordance with an embodiment of the invention;

Figure 7 is a representation of the stent of Figure 6 when viewed under the imaging system of Figure 1;

Figure 8 shows a microcoil in accordance with another embodiment of the invention;

Figure 9 is a partial view of the microcoil of Figure 8;

Figure 10 is a representation of a beam hardened artifact caused by a prior art microcoil when viewed under the imaging system of Figure 1;

Figure 11 is a representation of the microcoil of Figure 9 after insertion into a patient and when viewed under the imaging system of Figure 1;

Figure 12 is a representation of a beam hardened artifact caused by a prior art carotid stent when viewed under the imaging system of Figure 1;

Figure 13 is a representation of a carotid stent in accordance with another embodiment of the invention after the carotid stent has been inserted into a patient and when viewed under the imaging system of Figure 1;

Figure 14 shows a first embodiment of a stent delivery system;

Figure 15 shows the stent delivery system of Figure 14 upon inflation of a balloon;

Figure 16 shows a first embodiment of a sheath;

Figure 17 shows a second example of a sheath;

Figure 18 shows a third example of a sheath;

Figure 19 shows a second embodiment of a stent delivery system;

Figure 20 shows a third embodiment of a stent delivery system;

Figure 21A shows a fourth embodiment of a stent delivery system in a compressed state;

Figure 21B shows the fourth embodiment of the stent delivery system in an expanded state.

DETAILED DESCRIPTION OF THE DRAWINGS AND THE PRESENTLY PREFERRED EMBODIMENTS

It is therefore intended that the foregoing detailed description be regarded as illustrative rather than limiting, and that it be understood that it is the following claims, including all equivalents, that are intended to define the spirit and scope of this invention.

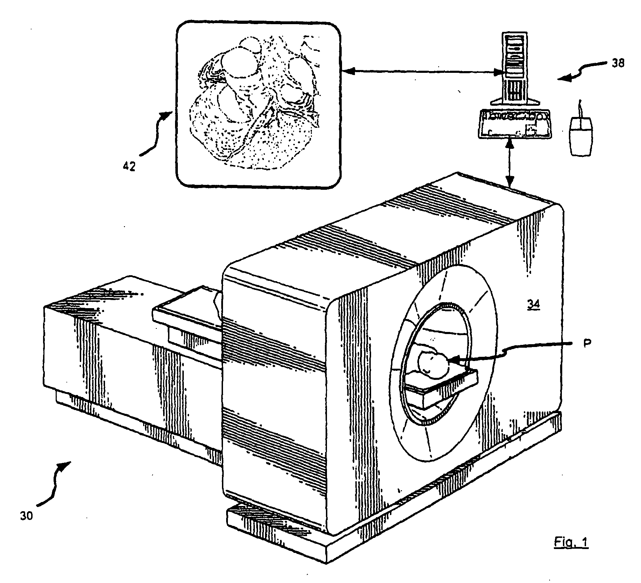

Referring now to Figure 1, an imaging system is indicated generally at 30. Imaging system 30 comprises a patient chamber 34, an image processing unit 38 and a display 42. Imaging system 30 can be based on any known or established imaging technology, but in a present embodiment is based on computed tomography (CT) having substantially the same functionality as a machine like the Toshiba Acquillon. Thus, patient chamber 34 is operable to capture images of a patient P in at least three planes, and processing unit 38 is operable to assemble those captured images to present a three-dimensional rendering of a target area within patient P on display 42. Images on display 42 can be navigated and/or viewed using the mouse and keyboard attached to processing unit 38, allowing the user to view a target area within patient P from any number of views. While not shown in Figure 1, image processing unit 38 can also be attached to other output devices in addition to display 42, such as a printer. Further, image processing unit 38 also typically includes a fixed storage device (such as a hard drive), a removable storage device (such as CD-Rewriter, or a tape drive) and a network interface card or other network interface means for connecting processing unit 38 to a network such as an intranet and/or the internet over which captured images can be delivered.

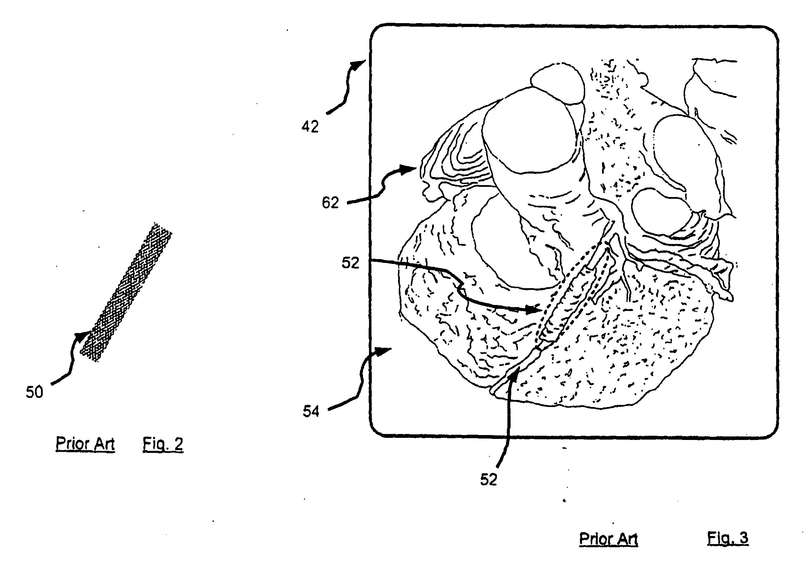

Referring now to Figure 2, a prior art conventional coronary stent is indicated at 50. Figure 2 shows stent 50 in isolation, however, for purposes of explaining the prior art, it is to be assumed that stent 50 has been implanted in a coronary artery of patent P.

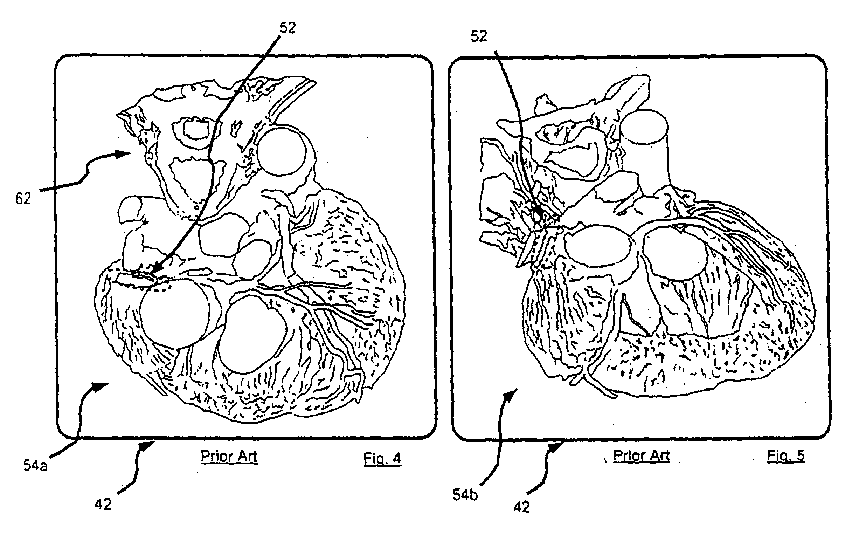

Figure 3 shows an image 54 rendered on display 42 of system 30 of patient P. Image 54 shows a beam hardened artifact52 as it is implanted inside a coronary artery 58 inside a heart 62 of patient P. The area identified as beam hardened artifact52 is an inaccurate reproduction of stent 50 as it is implanted inside artery 58. The beam hardening artifact52 is created by the material of stent 50. Accordingly, system 30 is of limited value in performing post-operative evaluations of stent 50 and for determining whether any restenosis has occurred of coronary artery 58.

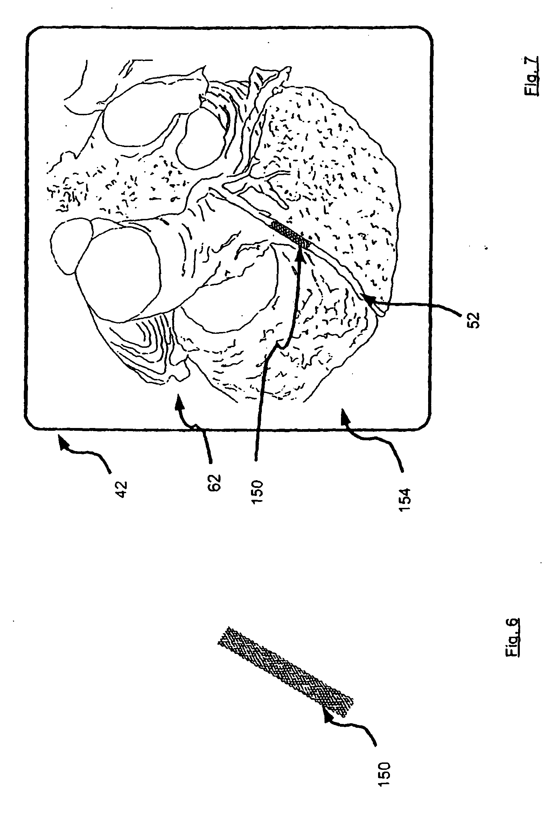

Figures 4 and 5 show additional images 54a and 54b, respectively, of different orientations of heart 62, which are readily produced on display 42 due to the imaging capability of system 30. In each image 54a and image 54b, stent 50 and the surrounding artery 58 are inaccurately reproduced due to beam hardening artifact52 of stent 50. Thus, notwithstanding the great flexibility of system 30 in being able to provide a multiplicity of views of heart 62, in its current form stent 50 and system 30 do not provide meaningful images for post-operative evaluation of artery 58 and the progress of any restenosis that may be occurring in the lumen of artery 58 surrounding stent 50.

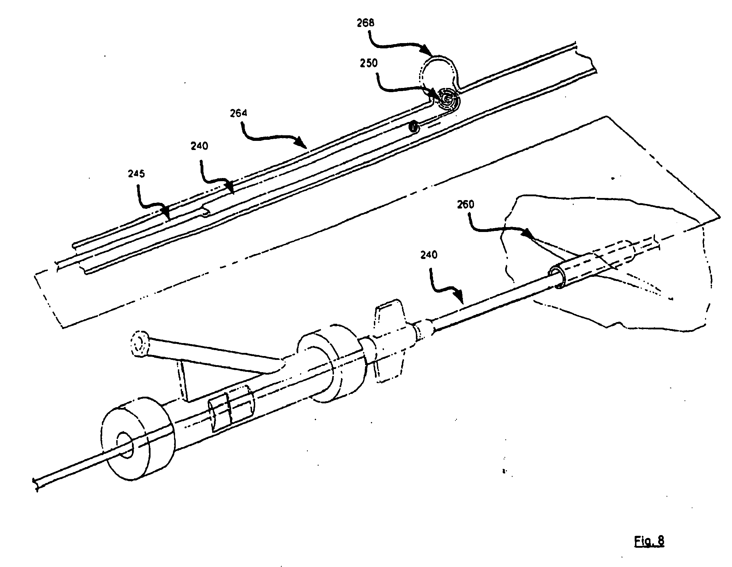

Figure 6 shows a medical device in accordance with an embodiment of the invention as a stent 150. Stent 150 from outward appearances is substantially the same as prior art stent 50, and indeed, in the present embodiment is designed to provide substantially the same mechanical and therapeutic functionality as prior art stent 50. However, in contrast to prior art stent 50, stent 150 is made from a material that has a selected radiopacity such that the appearance of stent 150 is preserved when stent 150 is exposed to the imaging beam of system 30 and presented on display 42. Thus, when stent 150 is implanted in heart 62, then in an image 154 of heart 62 generated by system 30, the appearance of stent 150 will be maintained when heart 62 and stent 150 are shown in display 42, as shown in Figure 7. Since image 154 has no beam hardened artefacts, it is now possible to examine the lumen of artery 58 surrounding stent 150, and thereby allow for an examination thereof for restenosis.

As will be appreciated by those of skill in the art, presence or absence of a beam hardening artifact can be measured according to the properties of the imaging system being used and in relation to the Hounsfield units associated with the particular material or tissue being exposed to the imaging beam. A relation between the linear attenuation coefficient ($\mu$) and the corresponding Hounsfield unit (H) can be expressed as:

$$H = \frac{\mu Material - \mu Water}{\mu Water} \, x1000$$

The value of the Hounsfield unit varies from -1000 (for air) to 1000 (for bone) to 3000, as more particularly shown in Table I.

Tissue Range of Hounsfield units Table I

| Material | Hounsfield Unit |
| --- | --- |
| Air | -1000 |
| Lung | -500 to -200 |
| Fat | -200 to -50 |
| Water | 0 |
| Blood | 25 |
| Muscle | 25 to 40 |
| Bone | 200 to 1000 |

The foregoing equation and table is found in *Principles of Computerized Tomographic Imaging Parallel CT, Fanbeam CT, Helical CT* and *Multislice CT* by Marjolein van der Glas, August 29, 2000, http://www.ph.tn.tudelft.nl/~marlein/pdf/CT.pdf.

Thus, in certain imaging systems materials with Hounsfield units exceeding about 1000 can be prone to creating beam hardening artifacts. Thus, presently preferred materials from which stent 150 can be manufactured to have reduced beam hardening artifacts include certain plastic, composite carbon fiber and Inconel metals that have similar mechanical properties to prior art stent 50 such that substantially the same therapeutic effect in stent 150 is achieved as was available in prior art stent 50. In any event, the chosen material for stent 150 has a level of Hounsfield density that diminish beam hardening artifacts to substantially preserve the appearance of the device under CT or other corresponding imaging beam.

It is thus presently preferred that stent 150 (or other medical devices according to the present invention) be made from a material or materials to have an overall image density of less than about 1200 Hounsfield Units. Such medical devices can also have an overall image density of less than about 900 Hounsfield Units. Such medical devices can also have an overall image density of less than about 700 Hounsfield Units. Such medical devices can also have an overall image density of less than about 400 Hounsfield Units.

As previously discussed, other medical devices are also within the scope of the present invention. The medical devices within the scope of the invention include devices for the treatment of obstruction due to clot, plaque, atheroma, tumours or the like, and/or treatments involving intimal hyperplasia and recurrent stenosis after stent placement. An appropriate device is delivered into the vascular or bilary system under image guidance. The post placement follow up of the lumen is enabled by the diminished density and beam hardening artifact of the construct and coating of the stent.

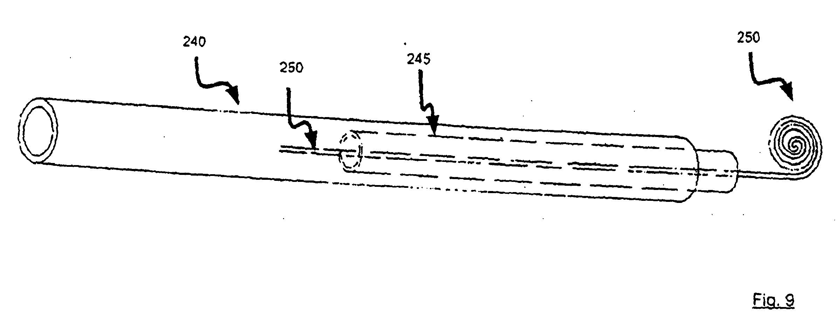

A specific example of another medical device within the scope of the invention is shown in Figures 8 and 9, which shows a microcoil 250 for treatment of an aneurysm and which is introduced via a guiding cathether 240 and a microcatheter 245. As best seen in Figure 8, guiding cathether 240 is inserted through an incision 260 near the femoral artery or brachial artery or other suitable location and passed through the venous system of the patient until it reaches a blood vessel 264 proximal to an aneurysm 268 in the patient's head. (Further discussion of this procedure can be found in the Inventor's copending application entitled "Method and Apparatus for Reducing Exposure to an Imaging Beam" and filed in the US Patent Office on March 3, 2003, the contents of which are incorporated herein by reference.)

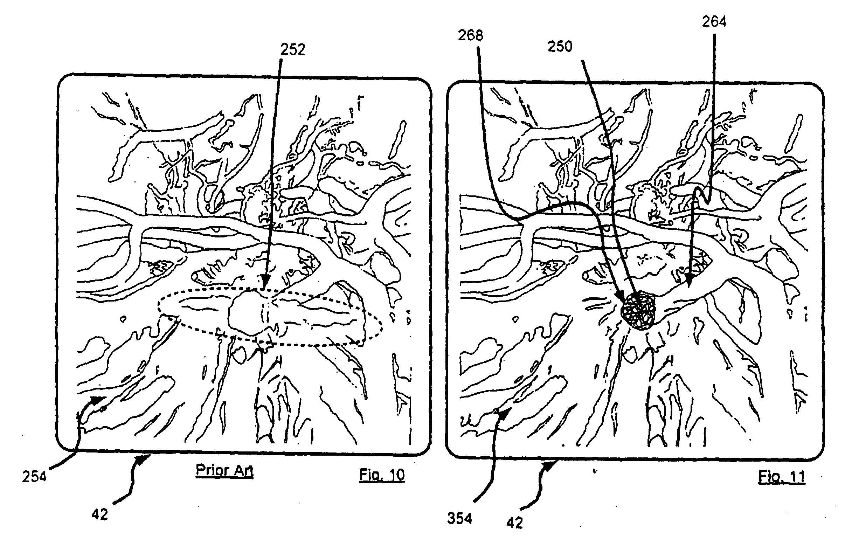

Figure 10 shows an image 254 of the resulting beam hardened artifact 252 when a prior art microcoil (not shown) is post-operatively examined using imaging system 30 has been previously inserted in the patient according to the method described in reference to Figure 8. The beam hardened artifact 252 thus renders it difficult, if not impossible, to accurately examine the prior art microcoil using imaging system 30.

However, as seen in image 354 shown in Figure 11, when microcoil 250 is inserted according to the method described with reference to Figure 8, then microcoil 250, the now-repaired aneuryism 268 and blood vessel 264 leading thereto are all visible on display 42 and therefore capable of post-operative evaluation.

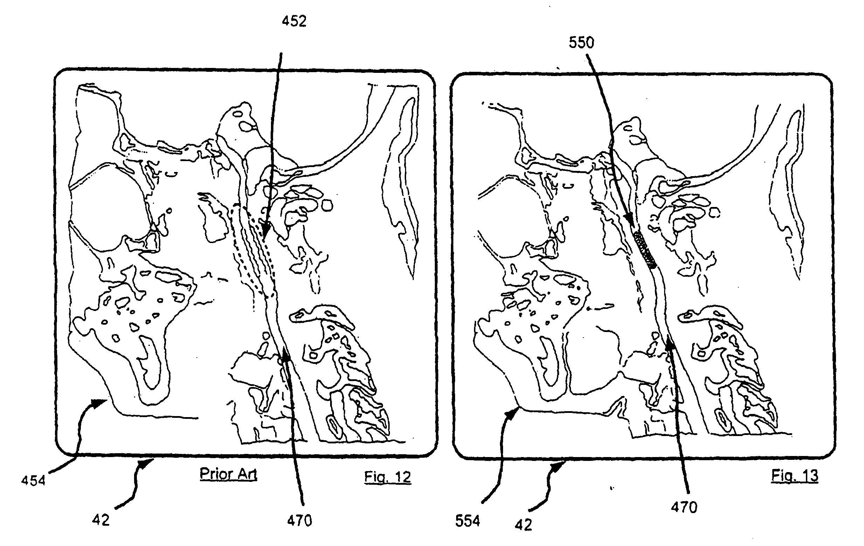

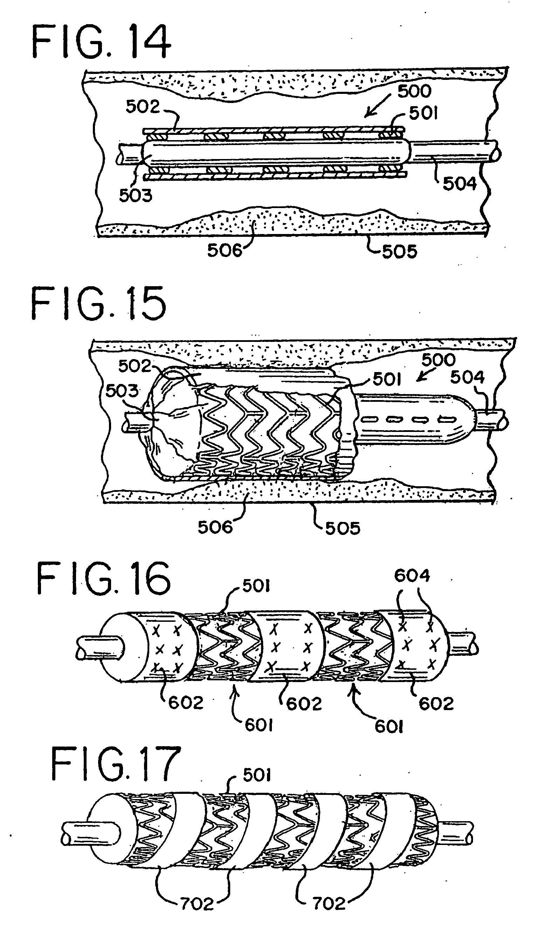

Another medical device within the scope of the invention is a carotid stent, for placement in the carotid artery. Figure 12 shows an image 454 of a sagittal view of patient along a plane that includes the carotid artery 470 of the patient. Image 454 is characterized by a beam hardened artifact 452 through which the lumen of an implanted prior art stent can be identified, but artifact 452 is severe enough to obscure the lumen of the carotid artery 470, therefore preventing a determination as to whether restenosis is occuring in the lumen of artery 470 surrounding the prior art stent. However, as shown in Figure 13, when a carotoid stent 550 in accordance with an embodiment of the present invention is used, stent 550 and the lumen of artery 470 surrounding the stent 550 can be viewed and the occurence of restenonis determined.

In other embodiments of the invention, the specific structure and/or configuration and/or shape of stent 150 (or other medical device) is chosen to further reduce the device's overall radiopacity. For example, the weave of the stent's structure can be chosen to reduce the radiopacity, and therefore the measured level of Hounsfield units associated with the stent.

Other aspects of the present invention provide a stent delivery system having a reduced number of passages of the stent or devices across the stenosis before dilating and deploying the stent in the stenosis. In certain prior art stent delivery systems, it is necessary to cross the wire, pre-dilate, and deploy the stent posteriorly. As a further example of a stent delivery system, a stent includes a self-expanding yet balloon mounted and intelligently be restrained. For example, the stent can be mounted on a balloon and deployed by inflation of the balloon. Such a stent is self-expanding but is delivered on the balloon. The inflation of the balloon breaks the restraining polymeric bands and results in the self-expansion of the stent once the initial stimulus has been given. This polymeric material is drug-coated and thrombosis resistant. This polymeric material helps restrain plaque and potential embolic material behind the stent. The overall configuration of the stent has reduced beam hardened artifacts post insertion when viewed under a CT system. Referring to Figures 14-20, various embodiments of the stent delivery system are described in detail below.

Figure 14 illustrates a cross sectional view of a stent delivery system 500. The stent delivery system 500 is delivered into a predetermined carotid artery 505 or other vessel being treated. The carotid artery 505 is located in a patient's neck. Stenosis 506 has developed on the wall of the carotid artery and the stent delivery system 500 is to be deployed to dilate the vessel 505. The stent delivery system 500 includes the stent 501 and the sheath 502. A balloon 503 mounted on the distal tip of a catheter 504 is surrounded by the stent 501 and the sheath 502. The balloon 503 referred to herein is typical of the type of balloon usually used with balloon-expandable stents. The catheter 504 is introduced into the blood vessel 505 with the stent 501, the sheath 502 and the balloon 503 mounted thereon.

The stent 501 is a self-expandable stent. When the stent 501 is used with a carotid artery, for example carotid artery 505, it is neither permanently deformed nor damaged by external trauma on the patient's neck. The stent 501 may be temporarily deformed by external traumas, but returns to the original shape as a result of its spring-like characteristics.

The stent 501 is delivered into the vessel 505 in a compressed state as shown in Fig. 14. The sheath 502 constrains the stent 501 in a collapsed state on the balloon tip of the catheter 504. Upon deployment, the balloon 503 is inflated to expand the stent 501 and the sheath 502 as illustrated in Fig. 15. Subsequently, the sheath 502 releases the stent 501 so that the stent 501 deploys into and supports the vessel 505. Accordingly, the sheath 502 is configured to fracture in order to release the stent 501. Once in the expanded state, the stent 501 provides structural support to the wall of the vessel 505.

Figures 16-18 illustrate various structures and designs of a sheath that is configured to fracture in order to release the stent 501. In Figure 16, the sheath is designed to include a plurality of bands 602. The bands 602 are arranged parallel to one another and spaced apart according to a longitudinal direction of the stent 501. The bands 602 constrain the stent 501 in the compressed state. Upon expansion of the stent 501, the bands 602 are configured to fracture to release the stent 501. When the stent 501 expands, the bands 602 are prone to fracture because they are unable to withstand the balloon pressure that arises during expansion of the stent 501. The bands 602 may be selected from an elastic material that has a predetermined threshold level of stretching. For example, the bands 602 may be made of various polymers compatible with human physiology. If the bands 602 are expanded above the threshold level, they are configured to fracture. As shown in Figure 16, at least one portion 601 of the stent 501 is exposed between the bands 602. This facilitates breaking or fracturing of the bands 602 because the stent 501 is less restricted by a sheath. In addition, other structures such as perforations may be added to encourage the fracturing of the bands 602. As shown in Figure 16, the bands 602 may be sutured to the struts of the stent 501. The sutures 604 prevent the bands 602 from drifting away from the stent 501 into the vessel 505 during the deployment.

Figure 17 depicts a plurality of strips 702 that surround the stent 501. The strips 702 are designed to have a narrower width than the bands 602. The strips 702 are arranged parallel to each other and angled relative to a longitudinal direction of the stent 501. The range of the angle of the strips 702 may be between 0° and 90°. Like the bands 602, the strips 702 constrain the stent 501 to keep the stent 501 in the compressed state. The strips 702 may be fractured upon expansion of the stent 501.

In one embodiment, wires may be used to restrict the stent 501 instead of the strips 702. The wires may be made from metal and may be selected from a group of metals compatible with human physiology. For example, stainless steel or nitinol may be used to form the wires. The wires may surround the stent 501 in a similar manner that the strips 702 restrict the stent 501 as shown in Figure 17. A certain portion of the wires may be thinner than the remaining portions so that each wire can be easily fractured upon inflation of the balloon 503. The wires as a constraining structure may be simple and convenient to design and manufacture.

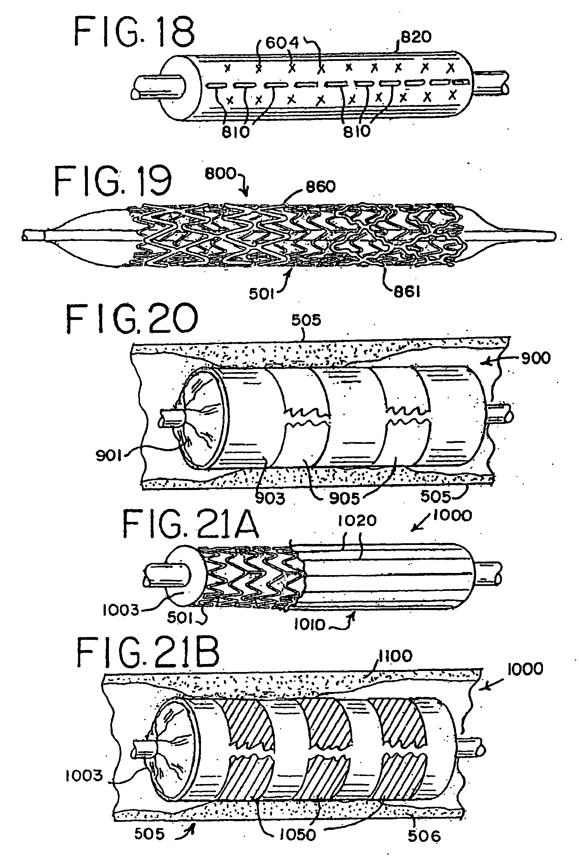

Figure 18 illustrates a sheath 820 that is continuous and that includes at least one perforation 810 formed on an outer surface thereof. Various shapes, sizes and designs of perforations are available. Due to the perforations 810, the sheath 820 can be easily fractured upon the expansion of the stent 501.

Referring to Figures 14-18, an operation of the stent delivery system 500 is explained as follows. The catheter 504 having a balloon mounted tip is introduced into the carotid vessel 505 developing stenosis 506. The self-expandable stent 501 in the compressed state and the sheath 502 constraining the stent 501 are disposed on the balloon mounted tip of the catheter 504. Preferably, no insertion of a balloon-tipped catheter or protection devices is performed either to predilate the vessel 505 or capture any embolic material prior to the insertion of the stent delivery system 500. Accordingly, the insertion of the stent delivery device 500 is the first passage into the host vessel 505.

To deploy the stent 501, the balloon 503 is inflated as shown in Figure 15. The stent 501 as well as the sheath 502 expand in response to the inflation of the balloon 503. After the sheath 502 is expanded to reach its threshold stretching level, it is fractured to release the stent 501. The sheath 502 may include the plurality of bands 602 or strips 702 as shown in Figures 16 and 17. Alternatively, the sheath 502 may have perforations such as perforations 810 shown in Figure 18. The bands 602, the strips 702 and the perforations 810 facilitate the fractures of the sheath 502 to release the stent 501. After fracturing, the sheath 502 remains captured between the expanded stent 501 and the vessel wall. In order to restrain the sheath and prevent it from being released into the blood vessel, sutures 604 may be provided to secure the sheath to the stent 501. Emboli captured between the sheath 502 and the vessel wall is also trapped along with the sheath 502. After the expansion of the stent 501 and the sheath 502, the balloon 503 is deflated and removed from the vessel by removing the catheter 504. The stent 501 remains within the vessel 505 and provides the structural support. Accordingly, the stent delivery system 500 passes into the vessel 505 only twice to deploy the stent 501 and remove the catheter 504 after the deployment of the stent 501. The stent delivery system 500 has the advantage that release of emboli and debris and damage to the vessel 505 arising out of passage of the stent delivery system 500, balloon 503 and protection device are substantially minimized.

Figure 19 illustrates a second embodiment of a stent delivery system 800. In this embodiment, balloon-expandable stents 860 and 861 are used as a sheath instead of the sheath 502, the bands 602, the strips 702 or the wires. In particular, the balloon-expandable stents 860, 861 are mounted on the self-expandable stent 501. In this embodiment, the balloon-expandable stents 860, 861 are different from each other, but two identical balloon-expandable stents may be used. Alternatively, a single, long balloon-expandable stent or three or more balloon-expandable stents may be used. As shown in Figure 14, the self-expandable stent 501 is mounted on the balloon 503. Accordingly, the stent delivery system 800 has a three layered structure, that is, the structure of the balloon 503, the self-expandable stent 501, and the balloon-expandable stent 860. The balloon-expandable stents 860, 861 are generally made from a ductile material, such as metal. For example, stainless steel or nitinol may be used, but any biocompatible metal is possibly used. When it is compressed, the balloon-expandable stents 860, 861 restrict the self-expandable stent 501 as a sheath as shown in Figure 19. As previously stated, because the balloon-expandable stents 860 and 861 are made from a ductile material, it maintains an initial, compressed state. Upon inflation of the balloon 503, the balloon-expandable stents 860, 861 are expanded along with the self-expandable stent 501. The balloon-expandable stents 860, 861 may also be designed to have minimal struts, which result in smaller or fewer openings between the struts. This design makes it possible for the balloon-expandable stents 860, 861 to effectively retain embolic material released from deployment of the stent 501. Various designs are possible for the balloon-expandable stents 860, 861 as long as such stents restrict the stent 501 in a compressed state and expands in response to the inflation of the balloon 503. One of the advantages of using a balloon-expandable stent as a sheath is that a balloon-expandable stent such as the balloon-expandable stents 860, 861 are longer in its collapsed form and shorten as they are expanded. This results in a larger space for a self-expandable stent such as the self-expandable stent 501 to be exposed. Further, a double stent area, i.e., where the self-expandable stent 501 and the balloon-expandable stents 860 and .861 are overlapped, is smaller.

Figure 20 shows a third embodiment of a stent delivery system. In Figure 14-18, a sheath constrains the stent 501 to keep the stent 501 compressed and is fractured to release the stent 501 when the stent 501 is expanded by the inflation of the balloon 503. In contrast, Figure 20 depicts another embodiment of a sheath used in the stent delivery system 900. In this embodiment, a sheath 903 does not constrain the stent 501. The sheath 903 is disposed on the stent 501 to trap debris behind the stent 501. The sheath 903 simply surrounds the stent 501 and does not impose any pressure on stent 501 to keep the stent 501 collapsed on the balloon 901. The sheath 903 is made of a non-elastic material, but it is plastically deformable and expands in response to the inflation of the balloon. Once the sheath 903 changes its shape, it is deformed and does not return to its original shape.

The balloon 901 is inflated to expand the stent 501. The sheath 903 expands along with the stent 501 as shown in Figure 20. After the expansion, the sheath 903 stays within the blood vessel 505 along with the stent 501 that provides the structural support to the vessel 505. The sheath 903 effectively traps debris released as a result of the stent deployment, thereby minimizing embolization and/or restenosis caused by such debris.

Instead of the sheath 903, the stent delivery device 900 includes another structure for constraining the stent 501. As shown in Figure 20, at least one constraining structure 905 constrains both the stent 501 and the sheath 903. The constraining structure 905 is fractured upon the inflation of the balloon 901 and releases both the stent 501 and the sheath 903. The constraining structure 905 includes at least one band 905 as illustrated in Figure 20, but other shapes and designs are possible. The constraining structure 905 is made of elastic material that has a predetermined threshold level of stretching. When the constraining structure 905 is expanded to exceed the threshold stretch level, it is configured to fracture. The fractured structure 905 is trapped between the vessel wall and the sheath 903 and does not drift within the vessel 505. Sutures that secure the constraining structure 905 to the stent may be included. Accordingly, the fractured structure 903 does not cause any embolization or restenosis.

The stent delivery system 900 operates as follows. The stent delivery system 900 is introduced into the vessel 505. Prior to insertion of the stent delivery system 900, no predilation of the vessel 505 by a balloon or an insertion of a protection device is performed. The stent 501. is restrained in the compressed state by the constraining structure 905. The balloon 901 is inflated to expand the stent 501. Along with the stent 501, the sheath 903 is expanded and plastically deformed. The constraining structure 905 is expanded to reach its threshold level and ultimately is fractured as shown in Figure 20. As a result, the debris or emboli released during the stent deployment is trapped between the sheath 903 and the vessel wall. The sheath 903 is trapped between the stent 501 and the vessel wall. Accordingly, embolic or debris release is substantially minimized even though no separate protection device is used. As a result, passages of the host vessel 505 for implanting the stent 501 are limited to two passes, thereby substantially minimizing embolic release and damage to the vessel arising out of such passages. Additionally, the sheath 903 continuously surrounds the expanded stent 501 throughout the deployment process, and therefore the vessel wall is protected from damage resulting from contact with the struts of the stent 501.

Figure 21 shows a fourth embodiment of a stent delivery system. A stent delivery system 1000 includes a sheath 1010 that is made of SIS material (Small Intestine Submucosa). Specifically, Figure 21A illustrates the stent delivery system 1000 when the stent 501 is in a compressed state, whereas Figure 21B illustrates that the stent 501 is expanded by inflation of a balloon 1003. The SIS material is derived from the small intestine of a pig and may be used to heal wounds or repair tissue damage. The SIS material is mechanically and chemically processed to be implanted into a patient's body. The SIS material typically has a matrix structure. When the SIS material is implanted, tissues adjacent to the SIS matrix begin to deliver cells and nutrients. Cells rapidly occupy the SIS matrix, and eventually, the SIS matrix is replaced by newly grown tissues. The SIS remains at the implantation site and provides support to the new tissues.

When the SIS material is used with a stent delivery system, it may repair the defect or lesion of the vessel walls. Specifically, the SIS material is used as a sheath covering a stent and the SIS material works as a smooth buffer between the stent and the vessel walls upon deployment of the stent. The stent is generally made of metal and upon expansion of the stent, the structure of the stent such as the struts may damage the vessel wall tissues. The SIS material is soft and thick, thereby protecting the vessel wall from damage. Tissues of the vessel wall grow into a matrix structure of the SIS sheath, thereby minimizing release of embolic materials such as necrotic tissues. In addition, the matrix structure of the SIS sheath is readily configured to embed at least one drug such as a restenosis inhibiting drug.

The SIS material does not readily stretch in response to the stent expansion. As shown in Figure 21A, a plurality of wrinkles 1020 are provided to make the sheath 1010 stretch in response to the expansion of the stent 501. In addition, the sheath 1010 does not constrain the stent 501. It is simply disposed on the stent 501. To constrain the stent 501, at least one constraining structure 1050 is provided in Figure 21B. The constraining structure 1050 restrains the stent 501 to be compressed. Upon expansion of the stent 501, the constraining structure 1050 is fractured and releases the stent 1001. As previously stated, the sheath 1010 remains with the stent 501 after the expansion, to enclose debris between the sheath 1010 and the vessel wall. The constraining structure 1050 is trapped between the sheath 1010 and the vessel 505. Sutures or other structures may be used to secure the sheath 1010 and constraining structure 1050 to the stent 1001.

As shown in Figures 20, 21A and 21B, the constraining structure 905, 1050 includes a plurality of bands. Alternatively or additionally, the constraining structure 905, 1050 may include strips and/or wires. Various shapes are possible and two or more shapes may be used together for the constraining structure 905, 1050. Further, the constraining structure 905, 1050 may be made from polymeric material, metal or any material that is compatible with human physiology.

An operation of the stent delivery system 1000 is performed in the same manner as the operation described in conjunction with Figure 20. Because the sheath 1010 is made of SIS material, the stent delivery system 1000 provides additional advantages such as healing any damage to the vessel wall.

The number of passages of hardware across the stent or devices across the stent is reduced from as many as five (as found in prior art delivery methods) to two passes (according to the described method passes). Thus, material that would otherwise become potentially an embolic source is restrained against the vessel wall.

This can be helpful in reducing the risks of stroke after carotid stenting and in some circumstances can reduce the need for distal flow protection devices which themselves have stroke risk.

In another variation of the present invention, shunt 150 is coated (either in its entirety or in particular locations) with an opacifier to temporarily increase the Hounsfield units associated with shunt 150 during its insertion, to allow shunt 150 to be inserted using traditional means. Such a coating would be configured to gradually abate and dissolve into the patient's blood stream, such that the radiopacity and associated Hounsfield units of stent 150 would decrease over time, such that under a post-operative CT evaluation, the Hounsfield units associated with stent 150 are low enough to allow proper visualization of the lumen of artery 58 surrounding stent 150. Suitable materials for coating shunt 150 include gold, iodine, ionic and non ionic iodinated compounds, ethiodol, and lipiodol, barium, tungsten, tantalum, gadolinium. Whatever coating is chosen, the amount and rate of dissolving of the coating is chosen to reduce toxicity experience by the patient during dissolution.

In a presently preferred embodiment, the aforementioned coating is a hydrophilic polymer containing a restenosis inhibiting drug and a density enhancing radiologic material such as lyophilizied iodinated contrast material, which is embedded into the polymer. This coating is then placed over a stent 150 that is made from a suitable material such as a plastic or metal, such as stainless steel, inconel or metal glass (materials already approved by The United States of America Food and Drug Administration), or an optimal arrangement of strands of another metal can be used. The result is that stent 150 is both drug eluting and density eluting (i.e. the level of Hounsfield units associated with the stent decreases over time.)

The stent 150 may be used in the stent delivery systems 500, 900 and 1000 described in conjunction with Figures 14-21. The stent 150 may be a self-expandable stent and disposed on the balloon mounted catheter like the stent 501. In Figures 14-21, because the stent 501 is a self-expandable stent, it is suitable for carotid arteries that are often subject to external traumas. The balloon is inflated to expand the stent 150 and the sheath 502, 903 and 1010. The sheath 502 is configured to be fractured in response to the expansion of the stent 150. In contrast, the sheaths 903 and 1010 expand and remain within the carotid artery 505 to trap the embolic release between the sheath 502, 903 and 1010 and the vessel wall. The sheath 903 is plastically deformable and the sheath 1010 is made of SIS material. Separate constraining structures 905 and 1050 are provided to constrain the stent 150 instead of the sheaths 903 and 1010.

In another embodiment of the invention, certain post processing software is provided in image processing unit 38 to maximize vascular conspicuity in conjunction with the known Hounsfield units and other imaging properties associated with stent 150 or other medical device in accordance with the present invention. For example, where a level of Hounsfield units associated with stent 150 is known, then upon detection by system 30 of an item within the patient at that particular level of Hounsfield units, then that information can be used to identify the item as stent 150 and then to further enhance the image of the surrounding vascular region based on the known imaging properties (i.e. radiopacity, structure, etc.) and using known signal processing an filtering techniques.

While only specific combinations of the various features and components of the present invention have been discussed herein, it will be apparent to those skilled in the art that desired subsets of the disclosed features and components and/or alternative combinations of these features and components can be utilized, as desired. For example, the stents, coils and other medical devices according to the present invention can be coated with a material to decrease the risk of infection and restenosis, using techniques and compounds described in EP0797988A2 and EP1155689A2 to Angiotech Pharmaceuticals Inc. of Canada, and the University of British Columbia.

The present invention also provides certain novel methods for evaluating cervical and intracranial vascular stents using CT, including MDCTA, that is reliable and low cost and then to use these techniques for long term evaluation and outcome analysis of stenting. Sensitivity and specificity can then be determined for MDCTA by comparison to conventional catheter angiogram results. The radiographic density of the stent, coil or other device can be altered to enhance CT, X Ray, Ultrasound and MRI visibility of the lumen. For the purpose of enhanced accuracy of CT diagnostic imaging beam hardening artifacts will be reduced and/or minimized. The devices in the present invention are in contrast to prior art devices that have been developed for conventional fluoroscopy guidance and thus are of a radiodensity or radiopacity that exceeds the needs of CT for clear visualization, this excess density creates unwanted beam hardening artifact.

Furthermore, the present invention allows for a relatively non-invasive means to visualize the lumen of a blood vessel surrounding a previously installed stent (or other site of an implanted medical device). Due to the reduced beam hardening artifacts of the stent, obscuration of the lumen is reduced. This results in the ability to visualize the lumen non-invasively as compared follow-ups conducted by invasive repeat catheter angiography, with its resultant risk of stroke, death and/or injury to an important vessel or to otherwise obscure a critical finding. CTA and CTP are relatively less invasive imaging modalities that have been shown to aid in the diagnosis and treatment of acute ischemic stroke. Both utilize highspeed spiral CT scanning and three-dimensional volumetric reconstruction software to create various types of images following injection of IV contrast solution. CTA can provide three-dimensional vascular delineation similar to other non-invasive techniques as well as visualization of adjacent non-vascular soft-tissue. CTA can also offer rapid volume acquisition, limited reconstruction artifact and scan completion during the period of peak intravascular contrast enhancement. Using CTA, it is often possible to see filling defect in a vessel as a result of contrast displacement by clot or thrombus. The sensitivity for detecting flow abnormality in vessels in the circle of Willis by CTA can be at least 89% when compared to digital subtraction angiography ("DSA"), and CTA does not carry the up to 5% risk of complication, and the up to 0.5% risk of permanent stroke that DSA has been shown to carry.

The above-described embodiments of the invention are intended to be examples of the present invention and alterations and modifications may be effected thereto, by those of skill in the art, without departing from the scope of the invention which is defined solely by the claims appended hereto.

## Claims

1. A stent delivery system, comprising:

   a self-expandable stent expanding from a compressed state to an expanded state,
   a sheath configured to surround the stent in the compressed state;
   a catheter adapted to deliver the stent and the sheath into a predetermined deployment site; and,
   a balloon mounted on a tip of the catheter and inflating to expand the stent; and,
   wherein the sheath is trapped between the stent and a vessel wall upon deployment of the stent.

2. The stent delivery system of claim 1, wherein the sheath constrains the stent in the compressed state.

3. The stent delivery system of claim 1, wherein the sheath is fractured by inflation of the balloon to release the stent in the expanded state.

4. The stent delivery system of claim 2, wherein the sheath is fractured by inflation of the balloon to release the stent in the expanded state.

5. The stent delivery system of claim 1, wherein the sheath includes at least one perforation having a predetermined shape formed thereon.

6. The stent delivery system of claim 5, wherein the perforation is fractured by the inflation of the balloon.

7. The stent delivery system of claim 1, wherein the sheath comprises one of a band, a strip, a wire and a combination thereof.

8. The stent delivery system of claim 7, wherein the one of the band, the strip, the wire and the combination is fractured by the inflation of the balloon.

9. The stent delivery system of claim 1, wherein the sheath is sutured to a portion of the self-expandable stent.

10. A stent delivery system, comprising:

a self-expandable stent expanding from a compressed state to an expanded state,
a balloon-expandable stent configured to constrain the stent in the compressed state;
a catheter adapted to deliver the stent and the sheath into a predetermined deployment site; and,
a balloon mounted on a tip of the catheter and inflating to expand the stent; and,
wherein the balloon-expandable stent is trapped between the stent and a vessel wall upon deployment of the stent.

11. A stent delivery system, comprising:

a self-expandable stent expanding from a compressed state to an expanded state;
a sheath enclosing the stent in the compressed state and being plastically deformable in response to the expansion of the stent;
a catheter delivering the stent and the sheath into a predetermined deployment site;
a balloon mounted on a tip of a catheter and inflating to expand the stent; and,
a structure constraining the stent to maintain the compressed state and attached to one of the stent, the sheath and a combination thereof.

12. The stent delivery system of claim 11, wherein the structure is configured to fracture by inflation of the balloon.

13. The stent delivery system of claim 11, wherein the structure includes a plurality of bands.

14. The stent delivery system of claim 11, wherein the constraining structure is sutured to a portion of the self-expandable stent.

15. The stent delivery system of claim 11, wherein the sheath is trapped between the stent and a vessel wall upon deployment of the stent.

16. The stent delivery system of claim 11, wherein the constraining structure is trapped between the stent and a vessel wall upon deployment of the stent.

17. The stent delivery system of claim 12, wherein the structure includes at least one perforation having a predetermined shape formed thereon.

18. The stent delivery system of claim 12, wherein the structure is sutured to a portion of the sheath.

19. The stent delivery system of claim 11, wherein the structure is made from metal.

20. The stent delivery system of claim 11, wherein the structure includes a plurality of wires.

21. The stent delivery system of claim 20, wherein a wire includes at least one point that is thinner in a thickness.

22. The stent delivery system of claim 11, wherein the sheath is a balloon-expandable stent and the sheath is expanded upon inflation of the balloon.

23. The stent delivery system of claim 11, wherein the stent is coated with at least one drug selected from a group consisting of a restenosis inhibiting drug and a thrombosis resistant drug.

24. The stent delivery system of claim 23, wherein the drug is selected from the group consisting of aspirin, plavix and paclitaxel.

25. The stent delivery system of claim 23, wherein the stent gradually elutes the drug into the deployment site.

26. The stent delivery system of claim 11, wherein the sheath is coated with at least one drug selected from a group consisting of a restenosis inhibiting drug and a thrombosis resistant drug.

27. The stent delivery system of claim 26, wherein the drug is selected from the group consisting of aspirin, plavix and paclitaxel.

28. The stent delivery system of claim 26, wherein the sheath gradually elutes the drug into the deployment site.

29. A stent delivery system, comprising:

a self-expandable stent expanding from a compressed state to an expanded state;
a sheath surrounding the stent and made of a SIS material;
a catheter delivering the stent and the sheath into a predetermined deployment site;
a balloon mounted on a tip of the catheter and inflating to expand the stent; and,
a structure constraining the stent to maintain the compressed state and attached to one of the stent, the sheath and a combination thereof.

30. The stent delivery system of claim 29, wherein the sheath includes at least one fold formed thereon to be expandable in response to inflation of the balloon.

31. The stent delivery system of claim 29, wherein the structure is configured to be fractured by inflation of the balloon.

32. The stent delivery system of claim 29, wherein the structure includes at least one perforation formed thereon.

33. The stent delivery system of claim 29, wherein the structure includes a plurality of strips.

34. The stent delivery system of claim 29, wherein the structure includes a plurality of bands that surround the sheath.

35. The stent delivery system of claim 29, wherein the structure includes a plurality of wires that surround the sheath.

36. The stent delivery system of claim 29, wherein the sheath remains in the deployment site and the catheter and the balloon are removed from the deployment site.

37. The stent delivery system of claim 29, wherein the structure remains in the deployment site and the catheter and the balloon are removed from the deployment site.

38. The stent delivery system of claim 29, wherein the deployment site is a carotid artery.

39. The stent delivery system of claim 29, wherein the constraining structure is sutured to a portion of the self-expandable stent.

40. The stent delivery system of claim 29, wherein the constraining structure is sutured to a portion of the sheath.

41. The stent delivery system of claim 29, wherein the constraining structure is made from metal.

42. The stent delivery system of claim 29, wherein the stent is coated with at least one drug selected from a group consisting of a restenosis inhibiting drug and a thrombosis resistant drug.

43. The stent delivery system of claim 42, wherein the drug is selected from the group consisting of aspirin, plavix and paclitaxel.

44. The stent delivery system of claim 42, wherein the stent gradually elutes the drug into the deployment site.

45. The stent delivery system of claim 29, wherein the sheath is coated with at least one drug selected from a group consisting of a restenosis inhibiting drug and a thrombosis resistant drug.

46. The stent delivery system of claim 45, wherein the drug is selected from the group consisting of aspirin, plavix and paclitaxel.

47. The stent delivery system of claim 45, wherein the sheath gradually elutes the drug into the deployment site.

48. A method of deploying a stent in a vessel, without using a distal protection device and without predilating the vessel, comprising:

   delivering a stent and a sheath into a predetermined deployment site with a catheter, wherein the stent is a self-expandable stent and configured to be surrounded by the sheath;
   inflating a balloon mounted on a tip of the catheter;
   expanding the stent to fracture the sheath;
   releasing the expanded stent into the deployment site; and,
   removing the catheter and the balloon from the deployment site.

49. The method of claim 48, further comprising:

   trapping the sheath between the expanded stent and a wall of the deployment site.

50. A method of deploying a stent in a vessel, without using a distal protection device and without predilating the vessel, comprising:

   delivering a stent and a sheath into a predetermined deployment site with a catheter, wherein the stent is a self-expandable stent and configured to be surrounded by the sheath made of a plastically deformable material, the stent constrained by a structure attached thereto to maintain a compressed state;
   inflating a balloon mounted on a tip of the catheter;
   expanding the stent to fracture the structure;
   releasing the expanded stent into the deployment site; and,
   removing the catheter and the balloon from the deployment site.

51. The method of claim 50, further comprising:

trapping the structure between the expanded stent and a wall of the deployment site.

52. A method of deploying a stent in a vessel, without using a distal protection device and without predilating the vessel, comprising:

delivering a stent and a sheath into a predetermined deployment site with a catheter, wherein the stent is a self-expandable stent and configured to be surrounded by the sheath and wherein the sheath is made of a SIS material, the stent being constrained by a structure attached thereto to maintain a compressed state;
inflating a balloon mounted on a tip of the catheter;
expanding the stent to fracture the structure;
expanding at least one fold of the sheath to expand the sheath;
releasing the expanded stent into the deployment site; and,
removing the catheter and the balloon from the deployment site.

53. The method of claim 52, further comprising:

trapping the structure between the expanded stent and a wall of the deployment site.

54. A medical device, comprising:

a self-expandable stent;
an expanding balloon mounted to the self-expandable stent; and,
a plurality of breakable polymeric bands surrounding the stent and the balloon and releasably restraining the self-expandable stent; and,
wherein the balloon breaks the bands when the balloon is inflated to thereby free the stent for deployment and the polymeric bands restrain plaque and potential embolic material from forming behind the stent.

Fig. 1

42

62

52

54

52

Prior Art     Fig. 3

50

Prior Art     Fig. 2

52

Prior Art

Fig. 5

42

54b

52

62

Prior Art

Fig. 4

42

54a

Fig. 7

52

42

62

150

154

Fig. 6

150

Fig. 8

Fig. 9

Fig. 11

Fig. 10

Prior Art

Fig. 13

Fig. 12

Prior Art

# FIG. 14

# FIG. 15

# FIG. 16

# FIG. 17

## FIG. 18

604    820

810    810

## FIG. 19

800    860

501    861

## FIG. 20

505

900

901

903    905    505

## FIG. 21A

1020    1000

1003    501    1010

## FIG. 21B

1100    1000

1003

505    1050    506

**European Patent Office**

**PARTIAL EUROPEAN SEARCH REPORT**

which under Rule 45 of the European Patent Convention shall be considered, for the purposes of subsequent proceedings, as the European search report

**Application Number**

EP 05 02 2438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/073286 A1 (ARMSTRONG JOSEPH P ET AL) 15 April 2004 (2004-04-15)<br><br>* the whole document *<br>----- | 1-12, 14-18, 23-28 | A61F2/06 |
| X | US 6 533 806 B1 (SULLIVAN JASON ET AL) 18 March 2003 (2003-03-18)<br><br><br>* the whole document *<br>----- | 1-8, 11-13, 15-17, 20-28,54 | |
| X | US 2002/052640 A1 (BIGUS STEVE ET AL) 2 May 2002 (2002-05-02)<br>* the whole document *<br>----- | 1-9,54 | |
| X | US 5 980 565 A (JAYARAMAN ET AL) 9 November 1999 (1999-11-09) | 1,2,7, 9-11,13, 15-17, 19,20,22 | |
| Y | * the whole document *<br>----- | 29-47 | TECHNICAL FIELDS SEARCHED (IPC) |
| X | US 6 179 878 B1 (DUERIG THOMAS ET AL) 30 January 2001 (2001-01-30)<br>* the whole document *<br>----- | 1,2,7,10 | A61F |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :


Claims searched incompletely :


Claims not searched :


Reason for the limitation of the search:

see sheet C

| Place of search | Date of completion of the search | Examiner |
|---|---|---|
| Munich | 25 January 2006 | Steiner, B |

EPO FORM 1503 03.82 (P04C07)

**European Patent Office**

**PARTIAL EUROPEAN SEARCH REPORT**

Application Number

EP 05 02 2438

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | US 2004/176833 A1 (PAVCNIK DUSAN ET AL) 9 September 2004 (2004-09-09) * the whole document * ----- | 29-47 | |
| A | US 6 352 561 B1 (LEOPOLD ERIC W ET AL) 5 March 2002 (2002-03-05) * the whole document * ----- | 1-47,54 | |

TECHNICAL FIELDS SEARCHED (IPC)

EPO FORM 1503 03.82 (P04C10)

**European Patent**
**Office**

**INCOMPLETE SEARCH**
**SHEET C**

Application Number

EP 05 02 2438

Claim(s) not searched:
    48-53

Reason for the limitation of the search (non-patentable invention(s)):

Article 52 (4) EPC - Method for treatment of the human or animal body by surgery

## ANNEX TO THE EUROPEAN SEARCH REPORT
## ON EUROPEAN PATENT APPLICATION NO.

EP 05 02 2438

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2006

| Patent document cited in search report | | Publication date | Patent family member(s) | | Publication date |
|---|---|---|---|---|---|
| US 2004073286 | A1 | 15-04-2004 | US | 2002099431 A1 | 25-07-2002 |
| US 6533806 | B1 | 18-03-2003 | AU | 4026601 A | 10-05-2001 |
| | | | CA | 2383501 A1 | 12-04-2001 |
| | | | DE | 60019332 D1 | 12-05-2005 |
| | | | EP | 1237502 A1 | 11-09-2002 |
| | | | WO | 0124733 A1 | 12-04-2001 |
| | | | US | 2003028237 A1 | 06-02-2003 |
| US 2002052640 | A1 | 02-05-2002 | NONE | | |
| US 5980565 | A | 09-11-1999 | CA | 2318943 A1 | 29-04-1999 |
| | | | EP | 0955955 A1 | 17-11-1999 |
| | | | JP | 2001506176 T | 15-05-2001 |
| | | | WO | 9920205 A1 | 29-04-1999 |
| | | | US | 6214040 B1 | 10-04-2001 |
| US 6179878 | B1 | 30-01-2001 | CA | 2218814 A1 | 22-04-1998 |
| | | | DE | 69733111 D1 | 02-06-2005 |
| | | | EP | 0839506 A1 | 06-05-1998 |
| | | | JP | 10179761 A | 07-07-1998 |
| | | | US | 6086610 A | 11-07-2000 |
| US 2004176833 | A1 | 09-09-2004 | NONE | | |
| US 6352561 | B1 | 05-03-2002 | AU | 740736 B2 | 15-11-2001 |
| | | | AU | 5458298 A | 17-07-1998 |
| | | | CA | 2275921 A1 | 02-07-1998 |
| | | | EP | 0971642 A1 | 19-01-2000 |
| | | | JP | 2001506902 T | 29-05-2001 |
| | | | JP | 2004305778 A | 04-11-2004 |
| | | | WO | 9827894 A1 | 02-07-1998 |
| | | | US | 2002029077 A1 | 07-03-2002 |

EPO FORM P0459

For more details about this annex : see Official Journal of the European Patent Office, No. 12/82